# EUROPEAN PATENT SPECIFICATION

(11) **EP 1 581 495 B1**
(45) Date of publication and mention of the grant of the patent: **18.04.2007**
(21) Application number: 03768468.5
(22) Date of filing: 18.12.2003
(51) Int. Cl.: C07D 211/26, A61K 31/451

(54) **4-aryl-4-(naphth-1-ylmethylamino)methyl-piperidine compounds and their use**
4-Aryl-4-(Naphth-1-ylmethylamino)methyl-Piperidin-Verbindungen und deren Verwendung
Composés de 4-aryl-4-(naphth-1-ylméthylamino)méthyl-pipéridine et leur utilisation

(30) Priority: 20.12.2002 US 435130 P
(43) Date of publication of application: 05.10.2005
(73) Proprietor: AstraZeneca AB, 151 85 Södertälje (SE)
(72) Inventor: BERNSTEIN, Peter,2AstraZeneca Wilmington, Wilmington, DE 19850-5437 (US); WARWICK, Paul, AstraZeneca Wilmington, Wilmington, DE 19850-5437 (US)
(74) Representative: Hallybone, Huw George
(86) International application number: PCT/SE2003/002004
(87) International publication number: WO 2004/056771

(56) References cited:
- STEVENSON ET AL.: '4,4-Disubstituted Piperidine High-Affinity NK1 Antagonists: Structure-Activity Relationships and in Vivo Activity' J. MED. CHEM. vol. 41, 1998, pages 4623 - 4635, XP002173109

## Description

### Field of the invention:

This invention relates to the treatment of diseases in which serotonin, Substance P or Neurokinin A are implicated, for example, in the treatment of disorders or conditions such as hypertension, depression, generalized anxiety disorder, phobias, posttraumatic stress syndrome, avoidant personality disorder, premature ejaculation, eating disorders, obesity, chemical dependencies, cluster headache, migraine, pain, Alzheimer's disease, obsessive-compulsive disorder, panic disorder, memory disorders, Parkinson's disease, endocrine disorders vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania and headache.

### Background:

The mammalian neurokinins are peptide neurotransmitters found in the peripheral and central nervous systems. The three principal neurokinins are Substance P (SP), Neurokinin A (NKA) and Neurokinin B (NKB). N-terminally extended forms of at least NKA are known. Three receptor types are known for the principal neurokinins. Based upon their relative selectivities for the neurokinins SP, NKA and NKB, the receptors are classified as neurokinin 1 (NK₁), neurokinin 2 (NK₂) and neurokinin 3 (NK₃) receptors, respectively. In the periphery, SP and NKA are localized in C-afferent sensory neurons, which neurons are characterized by non-myelinated nerve endings known as C-fibers, and are released by selective depolarization of these neurons, or selective stimulation of the C-fibers. C-Fibers are located in the airway epithelium, and the tachykinins are known to cause profound effects which clearly parallel many of the symptoms observed in asthmatics. The effects of release or introduction of tachykinins in mammalian airways include bronchoconstriction, increased microvascular permeability, vasodilation, increased mucus secretion and activation of mast cells. Neurokinin antagonists that interact with NK₁, NK₂ and NK₃ receptors, having different chemical structures have been described. Particularly international publications WO 98/07722, WO 96/39383 and WO 98/25617, and regional publications EP 428434, EP 474561, EP 515240 and EP 559538 disclose the preparation of a variety of chemical structures.

NK₁ activity is also implicated in depression and anxiety, mice with genetically altered NK₁ receptors have decreased anxiety related behavior (Santarelli, L., *et. al.,* Proc. Nat. Acad. Sci. (2001), 98, 1912) and NK₁ antagonists have been reported to be effective in an animal model of depression (Papp, M., *et. al.,* Behav. Brain Res. (2000), 115, 19).

Serotonin Selective Reuptake Inhibitors (SSRIs) are widely used for the treatment of major depressive disorder (MDD) and are considered well-tolerated and easily administered. SSRIs, however, have a delayed onset of action, are associated with undesirable side effects, such sexual dysfunction, and are ineffective in perhaps 30% of patients (M. J. Gitlin, MJ, J. Clin. Psych., 55, 406-413, 1994).

Compounds with dual action as NK₁ antagonists and serotonin reuptake inhibitors may, therefore provide a new class of antidepressants. Indeed, compounds combining NK₁ antagonism and serotonin reuptake inhibition have been described (Ryckmans, T., *et. al.,* Bioorg. Med. Chem. Lett. (2002), 12, 261)

Stevenson et al (J. Med. Chem. Vol. 41, 1998, pages 4623-4635) discloses 4-phenyl-4-arylmethoxymethyl-piperidines and derivatives thereof.

### Description of the Invention:

We have discovered novel piperidinyl amine derivatives having dual NK₁ antagonist activity and SSRI activity. Accordingly, this invention comprises such compounds, pharmaceutical compositions containing such compounds and methods of using such compounds to treat central nervous system (CNS) and other disorders.

Compound of the invention are those in accord with formula I: 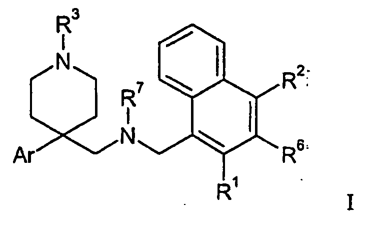 wherein:
R¹ and R² at each occurrence is independently selected from hydrogen, CN, CF₃, OCF₃, OCHF₂, halogen, C₁₋₄ alkyl, C₂₋₄ alkenyl, C₂₋₄ alkynyl, R^{a}, R^{b}, SR^{a}, NR^{a}R^{b}, CH₂NR^{a}R^{b}, OR^{c}, or CH₂OR^{c}, where R^{a}, R^{b}, and R^{c} are independently at each occurrence selected from hydrogen, C₁₋₆ alkyl, C(O)R^{d}, C(O)NHR^{d}, CO₂Rd, or R^{a} and R^{b} may together be (CH₂)ⱼG(CH₂)ₖ or G(CH₂)ⱼG where G is oxygen, j is 1, 2, 3 or 4, k is 0, 1 or 2; R^{d} at each occurrence is independently selected from C₁₋₆ alkyl;
R³ is hydrogen or C₁₋₄ alkyl;
R⁶ is hydrogen, CN, C₁₋₄ alkyl or C₁₋₄ alkoxy;
R⁷ is hydrogen or C₁₋₄ alkyl, and
Ar is phenyl or phenyl substituted at one or two positions with moieties independently selected from R⁴ or R⁵ where R⁴ and R⁵ are at each occurrence independently selected from halogen, C₁₋₄ alkoxy or halogenated C₁₋₄ alkyl;
and pharmaceutically-acceptable salts thereof. Particular compounds of the invention are those in accord with formula II, 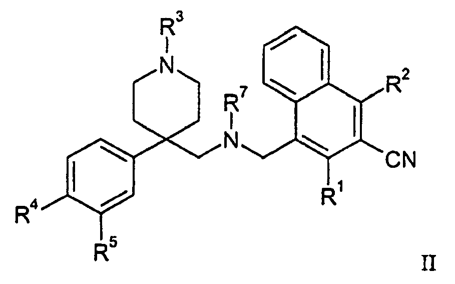 wherein:
R¹, R², R³, R⁴, R⁵ and R⁷ are as defined for formula I, in vivo-hydrolysable precursors thereof, and pharmaceutically-acceptable salts thereof.

Other compounds of the invention are those wherein:
Ar is selected from 4-chlorophenyl, 4-fluorophenyl, 4-methoxyphenyl, 3,4-dichlorophenyl, 3,4-dimethoxyphenyl, or 4-trifluoromethylphenyl, in vivo-hydrolysable precursors thereof, and pharmaceutically-acceptable salts thereof.

Yet further compounds of the inventions are those wherein
R¹ is selected from hydrogen, methoxy or ethyl;
R² is selected from hydrogen or methoxy,
R³ is selected from hydrogen or methyl; and pharmaceutically-acceptable salts thereof.

Another aspect of the invention is pharmaceutically-acceptable salts of a compounds as described herein made with an inorganic or organic acid which affords a physiologically-acceptable anion.

Particular pharmaceutically-acceptable salts of compounds of the invention are those wherein the inorganic or organic acid is selected from hydrochloric, hydrobromic, sulfuric, phosphoric, methanesulfonic, sulfamic, para-toluenesulfonic, acetic, citric, lactic, tartaric, malonic, fumaric, ethanesulfonic, benzenesulfonic, cyclohexylsulfamic, salicyclic or quinic acids.

Another aspect of the invention is a pharmaceutical composition comprising a compound of the invention or a pharmaceutically-acceptable salt thereof and a pharmaceutically-acceptable carrier.

Still another aspect of the invention is the use of a compound of the invention or a pharmaceutically-acceptable salt thereof in the preparation of a medicament for use in a disease condition selected from hypertension, depression in cancer patients, depression in Parkinson's patients, postmyocardial infarction depression, subsyndromal symptomatic depression, depression in infertile women, pediatric depression, major depression, single episode depression, recurrent depression, child abuse induced depression, post partum depression, generalized anxiety disorder, agoraphobia, social phobia, simple phobias, posttraumatic stress syndrome, avoidant personality disorder, premature ejaculation, anorexia nervosa, bulimia nervosa, obesity, addictions to alcohol, cocaine, heroin, phenobarbital, nicotine or benzodiazepines; cluster headache, migraine, pain, Alzheimer's disease, obsessive-compulsive disorder, panic disorder, dementia, amnestic disorders, age-related cognitive decline, dementia in Parkinson's disease, neuroleptic-induced parkinsonism, tardive dyskinesias, hyperprolactinaemia, vasospasm, cerebral vasculature vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania or headache associated with vascular disorders in a mammal, wherein antagonism of the NK₁ receptors and SSRI activity is beneficial.

In a particular aspect of the invention the use for treating a disorder or condition mentioned herein, comprises administering a compound of the invention in combination with a pharmaceutically-acceptable carrier.

Compounds in accord with formula I and pharmaceutically-acceptable salts may be made by processes as described and exemplified herein and by processes similar thereto and by processes known in the chemical art. If not commercially available, starting materials for these processes may be made by procedures which are selected from the chemical art using techniques which are similar or analogous to the synthesis of known compounds.

Pharmaceutically-acceptable salts may be prepared from the corresponding acid in a conventional manner. Non-pharmaceutically-acceptable salts may be useful as intermediates and as such are another aspect of the present invention.

It is well known in the art how to prepare optically-active forms (for example, by resolution of the racemic form or by synthesis from optically-active starting materials) and all optically active forms, enantiomers are compounds of this invention.

The following biological test methods, data and Examples serve to illustrate and further describe the invention.

The utility of a compound of the invention or a pharmaceutically-acceptable salt thereof (hereinafter, collectively referred to as a "Compound") may be demonstrated by standard tests and clinical studies, including those disclosed in the publications described below.

### Biological Assays:

### Test A: SERT Binding Assay:

Frozen membrane preparations of a stably transfected HEK293 cell line expressing human 5-HTT receptors were purchased from Receptor Biology (PerkinElmer). Frozen alliquots were rapidly thawed, homogenized, and diluted in assay buffer (AB) containing 50 mM TRIS-HCL, 120 mM NaCl, 5 mM KCl and adjusted to pH 7.4 with NaOH. Final protein concentration was 40 µg/ml. Test compounds were evaluated in competition assays utlilizing [³H]-Imipramine Hydrochloride purchased from NEN (PerkinElmer) as the radioligand. The stock radioligand was diluted with AB for a final concentration of approximately 2 nM. Kd for [³H]-Imipramine Hydrochloride was determined to be 2.7 nM. The competition assays were performed on 96-well assay plates - two drugs per plate. Ten serial dilutions (normally 1 µM to 38 pM final concentration) from stock 10 mM solutions of compounds prepared in DMSO. All serial dilutions were made using 20% DMSO. DMSO content in assay is less than 1%. Incubation mixtures were prepared in quadruplicate in 96-well plates (Costar). Final assay volumes per well were 10 µl compound/nonspecific/control (1% DMSO), 20 µl membranes, 20 µl [³H]-Imipramine Hydrochloride, and 150 µl AB. Specific binding was defined by using 10 µM Imipramine. The binding reaction was initiated by adding membranes immediately after adding the radioligand to wells containing buffer plus either test compound, nonspecific, or control. The assay plates were placed on a plate shaker and shaken for thirty minutes while the reactions reached equilibrium. The plates were then filtered through Beckman GF/B filters, presoaked in 6% PEI, using a Packard Filtermate 196. Filters were washed 5x with 0.2 ml ice-cold wash buffer (5 mM Tris HCl, pH 7.4.) The filters were dried and 35 µl of Microscint20 (Packard) was added to each well. The plates were then counted on a Packard TopCount to determine CPM's per well. Ki values were determined for each test compound utilizing the graphic and analytical software package, GraphPad Prism.

### Test B: NK₁ FLIPR Assay using Fluo-4 Dye:

FLIPR assays are performed with a device marketed by Molecular Devices, Inc., designed to precisely measure cellular fluorescence in a high throughput whole-cell assay. (Schroeder et. al., J. Biomolecular Screening, 1(2), p 75-80, 1996).

Compounds were evaluated for potency in blocking the response of U373 cells to the NK₁ receptor agonist Acetyl-[Arg⁶, Sar⁹, Met(O₂)¹¹]-Substance P (ASMSP) using a FLIPR instrument.

U373 cells were loaded with Fluo-4 dye (Molecular Probes) for 45 min at 37 °C and exposed to graded concentrations of compounds for 15 min at room temperature before being challenged with 10 nM - 12 nM ASMSP (an approximately EC₈₀ concentration). Responses were measured as the peak relative fluorescence after agonist addition. pIC₅₀s were calculated from eleven-point concentration-response curves for each compound.

### Reagents:

### Cell culture medium:

| | |
|---|---|
| Eagle's MEM with Earle's salts and 1-glutamine (500 mL) | Cellgro 10-010-CV |
| Non-essential amino acids, 100 x (5 mL) | Cellgro 25-025-CI |
| Sodium pyruvate, 100 mM (5 mL) | Cellgro 25-000-CI |
| L-Glutamine, 200 mM (5 mL) | Cellgro 25-005-CI |
| FBS (50 mL) | Cellgro 35-010-CV |

### Cell harvesting reagents:

| | |
|---|---|
| DPBS, 1x without Ca⁺⁺ & Mg⁺⁺ | Cellgro 21-031-CV |
| 1x Trypsin -EDTA (0.5% Trypsin, 0.53% EDTA-4Na) | Cellgro 25-052-CI |

### Cell plating medium:

| | |
|---|---|
| UltraCULTURE | BioWhittaker 12-725F |
| L-Glutamine, 200 mM (5 mL/500 mL) | Cellgro 25-005-CI |

### Working buffer:

| | |
|---|---|
| 10x Hank's balanced salt solution (100 mL/L) | Gibco 14065-056 |
| HEPES buffer 1 M (15 mL/L, [final] 15 mM) | Cellgro 25-060-CI |
| Probenecid (0.71 g dissolved in 6 mL 1 M NaOH for 1L, | |
| [final] 2.5 mM) | Sigma P-8761 |

DDH₂O to 1 L, adjust pH to 7.4 with NaOH

### Dye solution:

Fluo-4, AM dye, Molecular Probes F-14201. 50 µg lyophilized dye is dissolved in 23 µl DMSO plus 23 µL Pluronic F-127 (Molecular Probes P-3000). The 46 µL of solubilized fluo-4 dye is then added to 10 mL of working buffer solution to provide a working dye concentration of 5 µM. Each 10 mL of diluted dye is sufficient for a 384-well-plate of cells at 25 µL per well.

### Agonist:

Acetyl-[Arg⁶, Sar⁹, Met(O₂)¹¹]-Substance P (ASMSP)
Stock solution of 3.33x10⁻² M. Dissolve 100 mg in 3.05 mL DMSO and store in aliquots at 4 °C

### Miscellaneous:

### DMSO (to dissolve compounds and for tip wash)

### Cell culture and plating procedures:

U373 cells were grown in cell culture medium described above (30 mL per T-150 flask) and harvested when confluent as follows. Medium was removed by aspiration and cells were washed with 12 mL DPBS, 1x without Ca⁺⁺ and Mg⁺⁺. The DPBS was aspirated and replaced with 3 mL trypsin -EDTA. The cells plus trypsin/EDTA were incubated about 2 minutes at room temperature, until the cells detached from the flask. The harvesting reaction was quenched by addition of 9 mL culture medium and cells were resuspended by trituration. Cells were passaged at a transfer density of 1:4 every four days. For experiments, cells were counted, pelleted by centrifugation at 400 x g for 5 min and resuspended in cell plating medium at a density of 480,000 cells/mL. 25 µL of this cell suspension was added to each well of a black-walled 384-well plate (Falcon Microtest, 35 3962) using a Labsystems Multidrop 384 to give 12,000 cells per well. Plates were incubated at 37 °C overnight (minimum 15 h, maximum 23 h) before use.

### Compound and agonist preparation:

Compounds were dissolved in DMSO at a concentration of 10 mM and 120 µL of these solutions were transferred to the first well (column 1) of each row of a 96-well, roundbottomed, polypropylene storage plate (Costar 3365). Compounds on two such plates were then serially diluted simultaneously in DMSO using a Biomek 2000. 4 µL of each dilution was transferred to a deep well plate (Beckman Coulter 267006) which had been prepared previously to contain 400 µL of freshly made working buffer in each well. Concentrations resulting from this procedure are shown in Table 1. The final compound concentrations in the assay span 11 points, between 10 µM and 0.1 nM, in half-log increments.

The contents of the wells were mixed, and 45 µL of each dilution were transferred - in duplicate - to a 384-well polypropylene compound loading plate (Fisher 12-565-507) so that the 3 84-well plate contained duplicates of each of the compounds from both 96-well plates over the concentration ranges. Columns 23 & 24 of the plate contain no compound and serve as controls. Wells A - N in columns 23 and 24 were loaded with agonist only and therefore represent the maximal response. Wells O - P in columns 23 and 24 were loaded with only buffer, no agonist, and therefore represent the minimum response.

An ASMSP agonist loading plate was made by taking stock concentration of ASMSP and diluting in working buffer to give a concentration of 3.3 x 10⁻⁸ M. 45 µL of this solution were transferred to all wells of a 384-well polypropylene agonist loading plate (Fisher 12-565-507) except wells 023, 024; P23 & P24 which contained buffer alone and served as unstimulated controls.

### Dye Loading cells and adding compound:

For each 384-well assay plate of cells, 10 mL of diluted Fluo-4 dye was prepared as stated above in the methods/reagents section. First, each 384-well cell plate was washed once with working buffer on a CCS Packard plate washer. Any remaining post-wash buffer in the wells was removed by hand and 25 µL per well of Fluo-4 dye was added using a Labsystems Multidrop 384. The cell plate was returned to a 37 °C incubator for 45 min to allow the dye to permeate the cells. After 45 min of dye loading, the cell plates were washed twice with working buffer, leaving a 30 µL volume of buffer in each well. 5 µL of compound dilutions were transferred from the compound plate to the cell plate using a PlateMate. Assay plates were incubated in the presence of compound for 15 min at room temperature in the dark, and then loaded onto FLIPR.

### Recording responses in FLIPR:

After the 15 min compound pre-incubation, the plates were loaded onto the FLIPR instrument, 15 µL of ASMSP agonist was added and the cellular response to the agonist was recorded for 90 seconds. The response is measured as the peak relative fluorescence after agonist addition.

### Data analysis:

Results contained in the *stat* files generated by FLIPR were pasted into an Excel analysis template and, after outliers were excluded, IC₅₀ values were calculated within the template using XLfit. Individual IC₅₀ values were reported, along with pIC₅₀. When the two IC₅₀'s obtained for a compound differed by more than 3-fold that compound was assayed one or two more times to re-determine the value.

### Results:

Ki values obtained in the SERT assay for compounds of the invention ranged from less than 2 nM to about 180 nM. IC₅₀ values obtained in the FLIPR assay for compounds of the invention ranged from about 70 nM to about 2 µM.

### Examples:

The invention is illustrated by, but not limited to, the following examples in which descriptions, where applicable and unless otherwise stated, the following terms, abbreviations and conditions are used:

aq., aqueous; atm, atmospheric pressure; BOC, 1,1-dimethylethoxycarbonyl; DCM, dichloromethane; DMF, N,N-dimethylformamide; DMSO, dimethyl sulfoxide; EtOH, ethanol; Et2O, diethyl ether; EtOAc, ethyl acetate; h, hour(s); HPLC, high pressure liquid chromatography; HOBT, 1-hydroxybenzotriazole; MeOH, methanol; min, minutes; MS, mass spectrum; NMR, nuclear magnetic resonance; psi, pounds per square inch; RT, room temperature; sat., saturated; TEA, triethylamine; TFA, trifluoroacetic acid; THF, tetrahydrofuran.

Temperatures are given in degrees Celsius (°C); unless otherwise stated, operations were carried out at room or ambient temperature (18-25 °C).

Organic solutions were dried over anhydrous sodium or magnesium sulfate; evaporation of solvent was carried out using a rotary evaporator under reduced pressure (4.5-30 mm Hg) with a bath temperature of up to 60 °C.

Chromatography means flash column chromatography on silica gel unless otherwise noted; solvent mixture compositions are given as volume percentages or volume ratios.

When given, NMR data is in the form of delta values for major diagnostic protons (given in parts per million (ppm) relative to tetramethylsilane as an internal standard) determined at 300 MHz.

### Melting points are uncorrected.

Mass spectra (MS) were obtained using an automated system with atmospheric pressure chemical ionization (APCI) unless otherwise indicated. Masses corresponding to the major isotopic component, or the lowest mass for compounds with multiple masses with nearly equivalent abundance (isotope splitting), are reported.

Where noted that a final compound was converted to the citrate salt, the free base was dissolved in methanol, DCM, or acetonitrile, combined with citric acid (1.0 equivalents) in methanol, concentrated under reduced pressure and dried under vacuum (25-60 °C). When indicated that the salt was isolated by filtration from Et₂O, the citrate salt of the compound was stirred in Et₂O for 4-18 h, recovered by filtration, washed with Et₂O, and dried under vacuum (25-60 °C).

### Example 1: 1-N-Methyl-4-(4-chlororophenyl)-4-(3-(3-cyanonaphth-1-yl)-(2-azaprop-1-yl))piperidine.

The title compound of the following structure 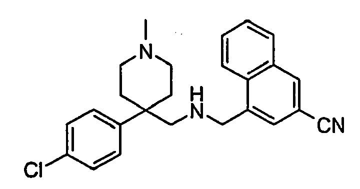 was prepared as a citrate salt as follows. To a cooled solution (0 °C.) containing 1-N-methyl-4-(4-chlorophenyl)-4-(aminomethyl)piperidine (0.100g, 0.42mmol) and triethylamine (0.114 mL, 0.82 mmol) in DMF (5 mL) was added a solution containing 3-cyano-1-iodomethyl naphthalene (0.100 g, 0.34 mmol) in DMF (2 mL) over a period of 5 min. The solution was stirred at 0 °C. for 30 min, then allowed to warm to room temperature overnight. The mixture was partitioned between ethyl acetate and saturated NaHCO₃, the organic layer was removed, and the aqueous layer extracted with ethyl acetate (2x). The organic extracts were washed with: 1) saturated NaHCO₃, 2) saturated brine, dried over MgSO₄, filtered, and concentrated. The residue was purified by chromatography (2-10% MeOH-DCM w/0.5% aq. NH₃) to give the title compound as a pale yellow solid (0.07g, 51 % yield). MS m/z 404.50 (M+H). The citrate salt was obtained by standard procedure.

The requisite 1-N-methyl-4-(4-chlorophenyl)-4-(aminomethyl)piperidine was prepared as follows:

### a) 1-N-Methyl-4-(4-chlorophenyl)-4-cyanopiperidine

To a solution containing mechlorethamine hydrochloride (1.93 g, 10 mmol) and 4-chlorophenyl acetonitrile (1.52 g, 10 mmol) in DMF (20 mL) was added sodium hydride (1.6 g, 40 mmol) portionwise at 0 °C. The resulting suspension was stirred and heated at 60 °C for 24 hrs. The reaction mixture was quenched with ice water, extracted with EtOAc (3x). The organic extracts were combined, washed with saturated NaCl (3x), dried, filtered, and concentrated. The residue was purified by chromatography (0-40% DCM-ETOAc) to give the title compound as a yellow oil (2.17 g, 92% yield). MS m/z 235.4 (M+H).

### b) 1-N-Methyl-4-(4-chlorophenyl)-4-(aminomethyl)piperidine

To a solution of 1-N-methyl-4-(4-chlorophenyl)-4-cyanopiperidine (0.95 g, 4.05 mmol) in dry THF (15 mL) was added LAH (1 M in THF, 12.15 mL, 12.15 mmol). The solution was stirred at room temperature for 2.5 hours. The reaction was quenched by adding water (2.5 mL), followed by 15% NaOH (2.5 mL) and water (2.5 mL). The mixture was then filtered through diatomaceous earth, washed with EtOAc, dried, filtered, and concentrated to give the title compound as a tan oil (0.85 g, 87% yield). MS m/z 239.5 (M+H).

The requisite 3-cyano-1-iodomethyl naphthalene was prepared as follows:

### c) 3-Cyano-1-hydroxymethyl naphthalene

To a cooled solution (0 °C.) containing DMF (3.75 mL, 50.8 mmol) in methylene chloride (60 mL) was added oxalyl chloride (12.7 mL, 145 mmol) dropwise. The resulting suspension was stirred at 0 °C for 1 hr. Solvent was removed under vacuum yielding a pale yellow solid. This solid was resuspended in acetonitrile (50 mL) and THF (100 mL) and cooled to 0 °C. To this cooled suspension was added a solution containing 3-cyanonaphthalene-1-carboxylic acid (7.5 g, 38.1 mmol) in THF (150 mL). The reaction was stirred at 0 °C for 1.5 hours then cooled to -78 °C. To this cooled solution was added, dropwise; a solution containing NaBH₄ (5.2 g, 137 mmol) in DMF (60 mL). The reaction was stirred at -78 °C for 1 hr, allowed to warm to -20 °C, held at -20 °C for 2 hr, then allowed to warm to room temp. Solvent was removed under vacuum.

The-residue was quenched with ice-cold aqueous HCl (1 N), extracted with EtOAc (2x). The organic extracts were combined, washed with: 1) HCl (1 N), 2) saturated NaCl (2x), dried, filtered, and concentrated. The residue was purified by chromatography (0-1% DCM-MeOH) to give the title compound as a yellow solid. (6.12 g, 88% yield). MS m/z fragments only.

### d) 3-Cyano-1-iodomethyl naphthalene

To a solution containing 3-cyano-1-hydroxymethyl naphthalene (5.85 g, 31.97 mmol) in acetonitrile (100 mL) under nitrogen was added trimethylsilylpolyphosphate (15 mL). Reaction was stirred at room temp for 15 minutes. To this solution was added NaI (8.3 g, 55.2 mmol). The suspension was stirred at room temperature overnight. Solvent was removed under vacuum. Residue was suspended in saturated NaHCO₃ (600 mL) and extracted with ethyl acetate (2x350 mL). Combined ethyl acetate extracts were washed with: 1) saturated NaHCO₃,2) saturated Na₂S₂O₃, 3) saturated brine, dried over MgSO₄, filtered, and concentrated. The residue was purified by crystallization from ethyl acetate yielding the title compound as a pale yellow solid (6.59g, 70% yield). MS m/z fragments only (M+H).

### Example 2: 1-N-Methyl-4-(4-fluorophenyl)-4-(3-(3-cyano-2-methoxynaphth-1-yl)-(2-azaprop-1-yl))piperidine.

The title compound of the following structure 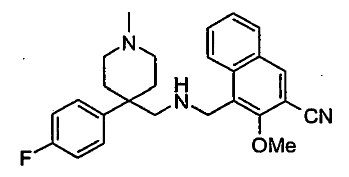 was prepared as a citrate salt in the same manner as Example 1, but using 1-N-methyl-4-(4-fluorophenyl)-4-(aminomethyl)piperidine (0.089 g, 0.40 mmol), triethylamine (0.114 mL, 0.82 mmol), 3-cyano-2-methoxy-1-iodomethyl naphthalene (110 mg, 0.34 mmol), DMF (7 mL), the title compound was obtained as a pale yellow solid. (55 mg, 39% yield, MS m/z 418(M+H).

The requisite 1-N-methyl-4-(4-fluorophenyl)-4-(aminomethyl)piperidine was prepared as follows:

### a) 1-N-Methyl-4-(4-fluorophenyl)-4-cyanopiperidine

To a solution containing mechlorethamine hydrochloride (1.923 g, 9.99 mmol) and 4-fluorophenyl acetonitrile (1.35 g, 9.99 mmol) in DMF (30 mL) was added sodium hydride (1.6 g, 40 mmol) slowly at 0 °C. The resulting suspension was stirred and heated at 60 °C for 24 hrs. The reaction mixture was quenched with ice water, extracted with EtOAc (3x). The organic extracts were combined, washed with saturated NaCl (3x), dried, filtered, and concentrated. The residue was purified by chromatography (2-5% MeOH-DCM) to give the title compound as a yellow oil (1.788 g, 82% yield). MS m/z 219.38 (M+H).

### b) 1-N-Methyl-4-(4-fluorophenyl)-4-(aminomethyl)piperidine

To a solution of 1-N-methyl-4-(4-fluorophenyl)-4-cyanopiperidine (1.788 g, 8.20 mmol) in dry THF (25 mL) was added LAH (1M in THF, 25 mL, 24.6 mmol). The solution was stirred at room temperature overnight. The reaction was quenched by adding water (2.5 mL), followed by 15% NaOH (2.5 mL) and water (2.5 mL). The mixture was then filtered through diatomaceous earth, washed with EtOAc, dried, filtered, and concentrated to give the title compound as a yellow oil (1.619 g, 89% yield). MS m/z 223.45 (M+H).

The requisite 3-cyano-2- methoxy-1-iodomethylnaphthalene was prepared as follows:

### c) 3-Cyano-2-methoxy-1-hydroxymethyl naphthalene

To a cooled solution (0 °C.) containing DMF (3.75 mL, 50.8 mmol) in methylene chloride (60 mL) was added oxalyl chloride (12.7 mL, 145 mmol) dropwise. The resulting suspension was stirred at 0 °C for 1 hr. Solvent was removed under vacuum yielding a pale yellow solid. This solid was resuspended in acetonitrile (50 mL) and THF (100 mL) and cooled to 0 °C. To this cooled suspension was added a solution containing 3-cyano-2-methoxynaphthalene-1-carboxylic acid (9.8 g, 38.1 mmol) in THF (150 mL). The reaction was stirred at 0 °C for 1.5 hours then cooled to -78 °C. To this cooled solution was added, dropwise; a solution containing NaBH₄ (5.2 g, 137 mmol) in DMF (60 mL). The reaction was stirred at -78 °C for 1 hr, allowed to warm to -20 °C, held at -20 °C for 2 hr, then allowed to warm to room temp. Solvent was removed under vacuum.
The residue was quenched with ice-cold aqueous HCl (1 N), extracted with EtOAc (2x). The organic extracts were combined, washed with: 1) HCl (1 N), 2) saturated NaCl (2x), dried, filtered, and concentrated. The residue was purified by chromatography (0-1% DCM-MeOH) to give the title compound as a yellow solid. (8.61 g, 93% yield). MS m/z fragments only.

### d) 3-Cyano-2-methoxy-1-iodomethyl naphthalene

To a solution containing 3-cyano-2-methoxy-1-hydroxymethylnaphthalene (2.20 g, 9.05 mmol) in acetonitrile (45 mL) under nitrogen was added trimethylsilylpolyphosphate (6 mL). Reaction was stirred at room temp for 15 minutes. To this solution was added NaI (1.7 g, 11.3 mmol). Reaction was stirred for 1.5 hr. Solvent was removed under vacuum. Residue was suspended in saturated NaHCO₃ (300 mL) and extracted with ethyl acetate (2x200 mL). Combined ethyl acetate extracts were washed with: 1) saturated NaHCO₃, 2) saturated Na₂S₂O₃ 3) saturated brine, dried over MgSO₄, filtered, and concentrated. The residue was purified by chromatography (3:1 DCM-hexane) to give the title compound as a yellow solid. (1.98 g, 63% yield). MS m/z fragments only.

### Example 3: 1-N-Methyl-4-(4-methoxyphenyl)-4-(3-(3-cyano-2,4-dimethoxynaphth-1-yl)-(2-azaprop-1-yl))piperidine.

The title compound of the following structure 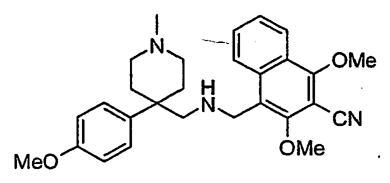 was prepared as a citrate salt in the same manner as Example 1, but using 1-N-methyl-4-(4-methoxyphenyl)-4-(aminomethyl)piperidine (0.089 g, 0.41 mmol), triethylamine (0.114 mL, 0.82 mmol), 3-cyano-2,4-dimethoxy-1-iodomethyl naphthalene (120 mg, 0.34 mmol), DMF (7 mL), the title compound was obtained as a pale yellow solid. (44 mg, 23% yield, MS m/z 460 (M+H).

The requisite 1-N-methyl-4-(4-methoxyphenyl)-4-(aminomethyl)piperidine was prepared as follows:

### a) 1-N-Methyl-4-(4-methoxyphenyl)-4-cyanopiperidine

To a solution containing mechlorethamine hydrochloride (1.93 g, 10 mmol) and 4-methoxyphenyl acetonitrile (1.48 g, 10 mmol) in DMF (20 mL) was added sodium hydride (1.6 g, 40 mmol) portionwise at 0 °C. The resulting suspension was stirred and heated at 60 °C for 24 hrs. The reaction mixture was quenched with ice water, extracted with EtOAc (3x). The organic extracts were combined, washed with saturated NaCl (3x), dried, filtered, and concentrated. The residue was purified by chromatography (0-40% DCM-ETOAc) to give the title compound as a yellow oil (2.32 g, 100% yield). MS m/z 231 (M+H).

### b) 1-N-Methyl-4-(4-methoxyphenyl)-4-(aminomethyl)piperidine

To a solution of 1-N-methyl-4-(4-methoxyphenyl)-4-cyanopiperidine (0.93 g, 4.05 mmol) in dry THF (15 mL) was added LAH (1 M in THF, 12.15 mL, 12.15 mmol). The solution was stirred at room temperature for 2.5 hours. The reaction was quenched by adding water (2.5 mL), followed by 15% NaOH (2.5 mL) and water (2.5 mL). The mixture was then filtered through diatomaceous earth, washed with EtOAc, dried, filtered, and concentrated to give the title compound as a tan oil (0.88 g, 93% yield). MS m/z 235(M+H).
The requisite 3-cyano-2,4-dimethoxy-1-iodomethylnaphthalene was prepared as follows:

### c) 3-Cyano-2,4-dimethoxy-1-hydroxymethylnaphthalene

To a cooled solution (0 °C.) containing DMF (3.75 mL, 50.8 mmol) in methylene chloride (60 mL) was added oxalyl chloride (12.7 mL, 145 mmol) dropwise. The resulting suspension was stirred at 0 °C for 1 hr. Solvent was removed under vacuum yielding a pale yellow solid. This solid was resuspended in acetonitrile (50mL) and THF (100mL). To this cooled suspension was added a solution containing 3-cyano-2,4-dimethoxynaphthalene-1-carboxylic acid (9.8 g, 38.1 mmol) in THF (150 mL). The reaction was stirred at 0 °C for 1.5 hours then cooled to-78 °C. To this cooled solution was added, dropwise; a solution containing NaBH₄ (5.2 g, 137 mmol) in DMF (60 mL). The reaction was stirred at -78 °C for 1 hr, allowed to warm to -20 °C, held at -20 °C for 2 hr, then allowed to warm to room temp. Solvent was removed under vacuum.
The residue was quenched with ice-cold aqueous HCl (1 N), extracted with EtOAc (2x). The organic extracts were combined, washed with: 1) HCl (1 N), 2) saturated NaCl (2x), dried, filtered, and concentrated. The residue was washed with hexane (3x) and dried under vacuum yielding the title compound as a white solid. (9.26 g, 100% yield). MS m/z fragments only.

### d) 3-cyano-2,4-dimethoxy-1-iodomethyl naphthalene

To a solution containing 3-cyano-2,4-dimethoxy-1-hydroxymethylnaphthalene (2.20 g, 9.05 mmol) in acetonitrile (45 mL) under nitrogen was added trimethylsilylpolyphosphate (6 mL). Reaction was stirred at room temp for 15 minutes. To this solution was added NaI (1.7 g, 11.3 mmol). Reaction was stirred for 1.5 hr. Solvent was removed under vacuum. Residue was suspended in saturated NaHCO₃ (300 mL) and extracted with ethyl acetate (2x200 mL). Combined ethyl acetate extracts were washed with: 1) saturated NaHCO₃, 2) saturated Na₂S₂O₃ 3) saturated brine, dried over MgSO₄, filtered, and concentrated. The residue was purified by chromatography (3:1 DCM-hexane) to give the title compound as a yellow solid. (1.98g, 63% yield). MS m/z fragments only.

### Example 4: 1-N-Methyl-4-(3,4-dichlorophenyl)-4-(3-(3-cyano-2 ethyl-naphth-1-yl)-(2-N-methyl-2-azaprop-1- yl))piperidine.

The title compound of the following structure 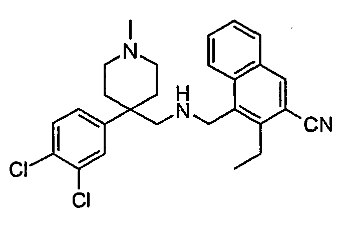 was prepared as a citrate salt in the same manner as Example 1, but using 1-N-methyl-4-(3,4-dichlororophenyl)-4-(aminomethyl)piperidine (0.093 g, 0.34 mmol), triethylamine (0.114 mL, 0.82 mmol), 3-cyano-2-ethyl-1-iodomethyl naphthalene (109 mg, 0.34 mmol), DMF (7 mL), the title compound was obtained as a pale yellow solid. (102 mg, 65% yield, MS m/z 466 (M+H).
The requisite 1-N-methyl-4-(3,4-dichlorophenyl)-4-(aminomethyl)piperidine was prepared as follows:

### a) 1-N-Methyl-4-(3,4-dichlororphenyl)-4-cyanopiperidine

To a cooled (ice bath) solution containing mechlorethamine hydrochloride (7.18 g, 37.3 mmol), 3,4-dichlorophenyl acetonitrile (6.97 g, 37.5 mmol), and dry DMF (100 mL) was added sodium hydride (60% dispersion in mineral oil) (6 g, 150 mmol) in portions over 10 min. The resulting suspension was heated at 60 °C for 24 h., then quenched with ice water and extracted with EtOAc (3x). The organic extracts were combined, washed with water (2X) and saturated NaCl (1X), dried, filtered, and concentrated. The residue was purified by chromatography (Silica Gel / 0-2% MeOH-DCM) and short-path distillation to give the title compound as a colorless oil (8.24 g, 82%) that solidified upon standing. MS m/z 269 (M+H).

### b) 1-N-Methyl-4-(3,4-dichlorophenyl)-4-(aminomethyl)piperidine

To a stirred solution containing 1-N-methyl-4-(3,4-dichlorophenyl)-4-cyanopiperidine (5.2 g, 19.3 mmol) and dry THF (20 mL) was added BH₃.THF (150 mL, 150 mmol). The solution was stirred and heated under reflux for 18 h., cooled (ice bath), carefully quenched with MeOH and 1 N aq. HCl, then concentrated *in vacuo.* The residue was treated with 8 N aq. HCl, the mixture heated under reflux for 18 h., then cooled and aq. NaOH and aq. NaHCO₃ added until basic. Following exhaustive extraction with DCM, the DCM extracts were combined, dried, filtered, and concentrated. The residual oil was purified by chromatography (Silica Gel / 0-5% MeOH-DCM w/ NH₄OH) and short-path distillation to give the title compound as a colorless oil (2.16 g, 41%). MS m/z 273 (M+H).
The requisite 3-cyano-2-ethyl-1-iodomethylnaphthalene was prepared as follows:

### c) 3-Cyano-2-ethyl-1-hydroxymethylnaphthalene

To a cooled solution (0 °C.) containing DMF (3.75 mL, 50.8 mmol) in methylene chloride (60 mL) was added oxalyl chloride (12.7 mL, 145 mmol) dropwise. The resulting suspension was stirred at 0 °C for 1 hr. Solvent was removed under vacuum yielding a pale yellow solid. This solid was resuspended in acetonitrile (50mL) and THF (100mL). To this cooled suspension was added a solution containing 3-cyano-2-ethylnaphthalene-1-carboxylic acid (8.57 g, 38.1 mmol) in THF (150 mL). The reaction was stirred at 0 °C for 1.5 hours then cooled to -78 °C. To this cooled solution was added, dropwise; a solution containing NaBH₄ (5.2 g, 137 mmol) in DMF (60 mL). The reaction was stirred at -78 °C for 1 hr, allowed to warm to -20 °C, held at -20 °C for 2 hr, then allowed to warm to room temp. Solvent was removed under vacuum. The residue was quenched with ice-cold aqueous HCl (1 N), extracted with EtOAc (2x). The organic extracts were combined, washed with: 1) HCl (1N), 2) saturated NaCl (2x), dried, filtered, and concentrated. The residue was purified by chromatography (95:5 DCM-EtOAc) to give the title compound as a white solid. (6.67 g, 83% yield). MS m/z 194 (M+H-H₂O).

### d) 3-Cyano-2-ethyl-1-iodomethylnaphthalene

To a solution containing 3-cyano-2-ethyl-1-hydroxymethyl naphthalene (1.50 g, 7.11 mmol) in acetonitrile (45 mL) under nitrogen was added trimethylsilylpolyphosphate (6 mL). Reaction was stirred at room temp for 15 minutes. To this solution was added NaI (1.92 g, 12.8 mmol). Reaction was stirred for 1.5 hr. Solvent was removed under vacuum. Residue was suspended in saturated NaHCO₃ (300 mL) and extracted with ethyl acetate (2x200 mL). Combined ethyl acetate extracts were washed with: 1) saturated NAHCO₃, 2) saturated brine, dried over MgSO₄, filtered, and concentrated. The residue was purified by chromatography (3:1 DCM-hexane) to give the title compound as a yellow solid. (1.96g, 86% yield). MS m/z. 196 (M+H-1).

### Examples 5-19:

Via reaction procedures similar to those given in Example 1-4 but with replacement of 4-chlorophenyl acetonitrile with the appropriately substituted phenyl acetonitrile, and with the replacement of 3-cyano-1-iodomethylnaphthalene with the appropriately substituted 3-cyano-1-iodomethylnaphthalene.

Examples and intermediates of Examples 1 to 23 are listed in Table 1.

**Table 1**

| | | | | | | | |
|---|---|---|---|---|---|---|---|
| 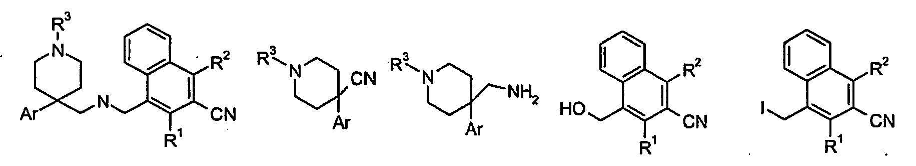 | | | | | | | |

| Example # | | Intermediate a) | Intermediate b) | | Intermediate c) | | Intermediate d) |
|---|---|---|---|---|---|---|---|
| **Ex. #** | **Ar** | | **R¹** | **R²** | **R³** | **Yield (%)** | **MS m/z (M+H)** |
| 1 | 4-chlorophenyl | | H | H | CH₃ | 51 | 404.5 |
| 1 a) | 4-chlorophenyl | | | | CH₃ | 92 | 235.4 |
| 1 b) | 4-chlorophenyl | | | | CH₃ | 87 | 239.5 |
| 1 c) | | | H | H | | 88 | No MH⁺ obs. |
| 1 d) | | | H | H | | 70 | No MH⁺ obs. |
| 2 | 4-fluorophenyl | | OCH₃ | H | CH₃ | 39 | 418 |
| 2 a) | 4-fluorophenyl | | | | CH₃ | 82 | 219.4 |
| 2 b) | 4-fluorophenyl | | | | CH₃ | 89 | 223.5 |
| 2 c) | | | OCH₃ | H | | 93 | No MH⁺ obs. |
| 2 d) | | | OCH₃ | H | | 63 | No MH⁺ obs. |
| 3 | 4-methoxyphenyl | | OCH₃ | OCH₃ | CH₃ | 23 | 460 |
| 3 a) | 4-methoxyphenyl | | | | CH₃ | 100 | 231.5 |
| 3 b) | 4-methoxyphenyl | | | | CH₃ | 93 | 235.5 |
| 3 c) | | | OCH₃ | OCH₃ | | 100 | No MH⁺ obs. |
| 3 d) | | | OCH₃ | OCH₃ | | 63 | No MH⁺ obs. |
| 4 | 3,4-dichlorophenyl | | CH₂CH₃ | H | CH₃ | 65 | 466 |
| 4 a) | 3,4-dichlorophenyl | | | | CH₃ | 82 | 269 |
| 4 b) | 3,4-dichlorophenyl | | | | CH₃ | 41 | 273 |
| 4 c) | | | CH₂CH₃ | H | | 83 | 194 (-H₂0) |
| 4 d) | | | CH₂CH₃ | H | | 86 | 196 (-I) |
| 5 | 4-chlorophenyl | | OCH₃ | H | CH₃ | 41 | 434.4 |
| 6 | 4-fluorophenyl | | H | H | CH₃ | 33 | 388 |
| 7 | 4-methoxyphenyl | | H | H | CH₃ | 30 | 400 |
| 8 | 4-methoxyphenyl | | OCH₃ | H | CH₃ | 33 | 430 |
| 9 | 3,4-dichlorophenyl | | H | H | CH₃ | 32 | 439 |
| 10 | 3,4-dichlorophenyl | | OCH₃ | H | CH₃ | 38 | 469 |
| 11 | 4-chlorophenyl | | OCH₃ | OCH₃ | CH₃ | 31 | 464.5 |
| 12 | 4-chlorophenyl | | CH₂CH₃ | H | CH₃ | 68 | 432.5 |
| 13 | 4-fluorophenyl | | OCH₃ | OCH₃ | CH₃ | 24 | 449 |
| 14 | 4-fluorophenyl | | CH₂CH₃ | H | CH₃ | 66 | 416 |
| 15 | 4-methoxyphenyl | | CH₂CH₃ | H | CH₃ | 62 | 428 |
| 16 | 3,4-dichlorophenyl | | OCH₃ | OCH₃ | CH₃ | 33 | 499 |
| 17 | 3,4-dimethoxyphenyl^{1a} | | H | H | CH₃ | 38 | 430 |
| 17 a) | 3,4-dimethoxyphenyl^{1b} | | | | CH₃ | 92 | 261.5 |
| 17 b) | 3,4-dimethoxyphenyl^{1c} | | | | CH₃ | 86 | 265.5 |
| 18 | 3,4-dichlorophenyl | | H | H | t-BOC^{2a} | 61 | 524 |
| 18 a) | 3,4-dichlorophenyl | | | | t-BOC ^{2b} | 54 | 255 (-BOC) |
| 18 b) | 3,4-dichlorophenyl | | | | t-BOC ^{2c} | 85 | 344 (-CH₃) |
| 19 | 3,4-dichlorophenyl | | H | H | H³ | 100 | 425 |
| 20 | 4-trifluoromethylphenyl | | H | H | CH₃ | 39 | 438 |
| 20 a) | 4-trifluoromethylphenyl^{1b} | | | | CH₃ | 79 | 269 |
| 20 b) | 4-trifluoromethylphenyl^{1c} | | | | CH₃ | 55 | 273.5 |
| 21 | 4-trifluoromethylphenyl | | OCH₃ | H | CH₃ | 51 | 468 |
| 22 | 4-trifluoromethylphenyl | | OCH₃ | OCH₃ | CH₃ | 48 | 498 |
| 23 | 4-trifluoromethylphenyl | | CH₂CH₃ | H | CH₃ | 94 | 466 |

| | | | | | | | |
|---|---|---|---|---|---|---|---|
| 1a, 2a. Prepared as described in Example 1. 1b. Prepared as described in Example 1a. 1c. Prepared as described in Example 1b. 2b. A solution containing bis(2-chloroethyl)-N-BOC amine (8.15 g, 33.67 mmol), 3,4-dichlorophenylacetonitrile (5.05 g, 27.17 mmol), and DMSO (50 mL) was stirred at RT and solid cesium carbonate (17.6 g, 54.02 mmol) was added (in portions) over 10 minutes. After 20 h, additional cesium carbonate (1.7 g,) was added, and the mixture stirred for an additional 72 h. The mixture was partitioned between water and EtOAc, the aqueous layer was removed, and the organic layer washed sequentially with additional water, 0.1 M aq. HCl (2X), sat. aq. NaHCO₃, and brine. The organic layer was dried, filtered, concentrated, and the residue triturated (3:1 hexane/ethyl acetate) to give the title compound (5.26 g, 54%) as an off-white solid, m.p. 142-145 °C. MS m/z 255 (M-BOC). 2c. A mixture containing 1-N-BOC-4-(3,4-dichlorophenyl)-4-cyanopiperidine (5.25 g, 14.78 mmol), Raney Ni catalyst (5 g of 50% aq. slurry), EtOH (175 mL), and ammonium hydroxide (88 mL) was placed under a hydrogen atmosphere (50 psi) and agitated (Parr apparatus) for 18 h. The mixture was filtered (diatomaceous earth), concentrated, and purified by chromatography (0-5% MeOH/DCM) to give the title compound as an off-white solid (4.53 g, 85%). MS m/z 344 (M-CH₃). 3. Prepared from the compound of Example 18 as follows: To a solution containing the compound of Example 18 (81mg, 0.155 mmol) in DCM (10 mL) was added TFA (3 mL) in 3x1 mL portions. Reaction was stirred at room temp for 30 min. Solvent was removed. Residue was suspended in saturated NaHCO₃, extracted with ethyl acetate (2x 100ml). Combined organic extracts were washed with: 1) saturated NaHCO₃ (2x35 mL), 2) saturated NaCl (2x35 mL), dried over MgSO₄, filtered, and solvent removed yielding the title compound as a white solid. (66 mg, 100% yield). MS m/z. 425 (M+H). | | | | | | | |

### Example 24: 1-N-Methyl-4-(4-chlororophenyl)-4-(3-(3-cyanonaphth-1-yl)-(2-N-methyl -2-azaprop-1-yl))piperidine.

The title compound of the following structure 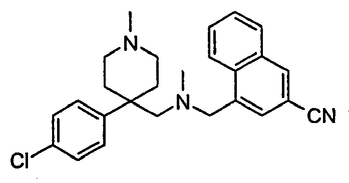 was prepared as a citrate salt as follows. To 1-N-Methyl-4-(4-chlororophenyl)-4-(3-(3-cyanonaphth-1-yl)-(2-azaprop-1-yl))piperidine (Example 1, 0.060 g, 0.42 mmol) was added formaldehyde (37% aqueous, 2.0 mL, 26.7 mmol) then formic acid (0.25 mL, 6.5 mmol). The suspension was stirred & heated at 100 °C. for 24 hours. The reaction was cooled to room temp, and solvent was removed under vacuum. The residue was partitioned between ethyl acetate and saturated NaHCO₃, the organic layer was removed, and the aqueous layer extracted with ethyl acetate (2x). The organic extracts were washed with: 1) saturated NaHCO₃, 2) saturated brine, dried over MgSO₄, filtered, and concentrated. The residue was purified by SFC chromatography (20-35% MeOH-CO₂ w / 0.5% (CH₃)₂NCH₂CH₃) to give the title compound as a pale orange solid (32 mg, 48% yield). MS m/z 417.50 (M+H). The citrate salt was obtained by standard procedure.

### Example 25: 1-N-Methyl-4-(4-fluororophenyl)-4-(3-(3-cyano-2-methoxynaphth-1-yl)-(2-N-Ethyl -2-azaprop-1-yl))piperidine.

The title compound of the following structure 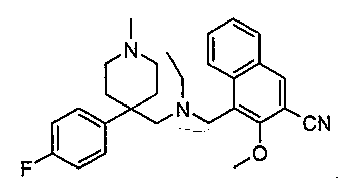 was prepared as a citrate salt as follows. To a solution containing 1-N-Methyl-4-(4-fluororophenyl)-4-(3-(3-cyano-2-methoxynaphth-1-yl)-(2-azaprop-1-yl))piperidine (Example 2, 0.050 g, 0.12 mmol) in THF (1.0 mL) was added acetaldehyde (8.4 uL, 0.30 mmol). Reaction was stirred for 5.0 min. To this solution was added MP-Triacetoxyborohydride(0.15 g, 0.3 mmol). The suspension was stirred for 18 hours. Resin beads were removed by decantation and solvent was removed under vacuum. The residue was purified by silica gel chromatography (4-10% MeOH-DCM w/0.5% aq. NH₃) to give the title compound as a pale tan film (0.046g, 86% yield). MS m/z 446 (M+H). The citrate salt was obtained by standard procedure.

### Example 26: 1-N-Methyl-4-(4-fluororonhenyl)-4-(3-(3-cyanonaphth-1-)-(2-N-Ethyl-2-azaprop-1-yl))piperidine.

The title compound of the following structure 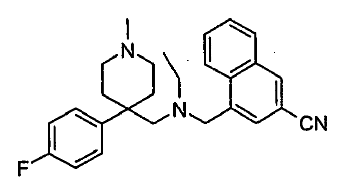 was prepared as a citrate salt as follows. To a solution containing 1-N-Methyl-4-(4-fluororophenyl)-4-(3-(3-cyanonaphth-1-yl)-(2-azaprop-1-yl))piperidine (Example 6, 0.050 g, 0.129 mmol) in THF (1.0 mL) was added acetaldehyde (8.4 uL, 0.30 mmol). Reaction was stirred for 5.0 min. To this solution was added MP-Triacetoxyborohydride(0.15 g, 0.3 mmol). The suspension was stirred for 18 hours. Resin beads were removed by decantation and solvent was removed under vacuum. The residue was purified by silica gel chromatography (4-10% MeOH-DCM w/0.5% aq. NH₃) to give the title compound as a pale tan film (0.03 9g, 72% yield). MS m/z 416 (M+H). The citrate salt was obtained by standard procedure.

### Examples 27-33:

Via reaction procedures similar to those given in Example 24, compounds of the following formula listed in Table 2 were obtained.

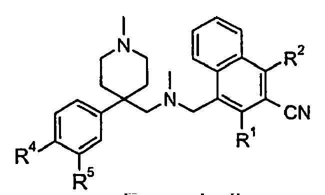

### Example #

**Table 2**

| **Example** # | **Starting material** | **R¹** | **R²** | **R⁴** | **R⁵** | **Yield (%)** | **MS m/z (M+H)** |
|---|---|---|---|---|---|---|---|
| 20 | Example 1 | H | H | Cl | H | 48 | 418.5 |
| 27 | Example 5 | OCH₃ | H | Cl | H | 33 | 448.5 |
| 28 | Example 6 | H | H | F | H | 57 | 402.5 |
| 29 | Example 2 | OCH₃ | H | F | H | 54 | 432.5 |
| 30 | Example 7 | H | H | OCH₃ | H | 54 | 414.5 |
| 31 | Example 8 | OCH₃ | H | OCH₃ | H | 50 | 444.2 |
| 32 | Example 9 | H | H | Cl | Cl | 58 | 452.1 |
| 33 | Example 10 | OCH₃ | H | Cl | Cl | 63 | 482.1 |

### Example 34: 4-(4-Fluorophenyl)-4-(3-(3-cyanonaphth-1-yl)-(2-azaprop-1-yl))piperidine.

The title compound of the following structure 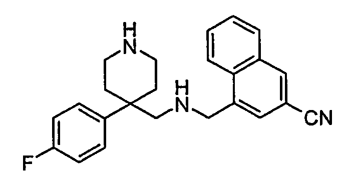 was prepared as a citrate salt as follows. To a solution of 1-N-Boc-4-(4-fluorophenyl)-4-(3-(3-cyanonaphth-1-yl)-(2-azaprop-1-yl))piperidine (108mg, 0.228mmol) in DCM (2 mL) was added trifluoroacetic acid (0.35mL, 4.56mmol). The solution was stirred at room temperature for 2 hours. Solvent was evaporated, EtOAc and saturated NaHCO₃ was added. The organic layer was then washed with saturated NaCl, dried over MgSO₄, filtered and concentrated to give the title product as a foaming light yellow solid (74mg, 87% yield). MS m/z 374.57 (M+H). The citrate salt was obtained by standard procedure.

The requisite 1-N-Boc-4-(4-fluorophenyl)-4-(3-(3-cyanonaphth-1-yl)-(2-azaprop-1-yl))piperidine was prepared as follows:

### a) 4-(4-Fluorophenyl)-4-cyanopiperidine

To a solution containing bis-2-chloroethylamine hydrochloride (4.00g, 22.4mmol) and 4-fluorophenyl acetonitrile (3.03g, 22.4mmol) in DMF (100mL) was added sodium hydride (3.6g, 89.6mmol) portionwise at 0°C. The resulting suspension was stirred and heated at 60°C for 24 hrs. The reaction mixture was quenched with ice water, extracted with EtOAc (3x). The organic extracts were combined, washed with saturated NaCl (3x), dried, filtered, and concentrated. The residue was purified by chromatography (0-10% MeOH-DCM) to give the title compound as a yellow oil (3.63g). MS m/z 205.3 (M+H)

### b) 1-N-Boc-4-(4-fluorophenyl)-4-cyanopiperidine

To a solution of 4-(4-fluorophenyl)-4-cyanopiperidine (3.63g, 17.8mmol) in THF (90mL) was added Boc-anhydride (3.88g, 17.8mmol) followed by diisopropylethylamine (3.1mL, 17.8mmol). The resulting solution was stirred at room temperature for 24 hrs. The reaction mixture was quenched with 0.1N HCl, extracted with EtOAc (3x). The organic extracts were combined, dried, filtered, and concentrated. The residue was purified by chromatography (0-1% MeOH-DCM) to give the title compound as a yellow oil (4.64g, 68% yield 2 steps). MS m/z 205.26 (M+H-Boc)

### c) 1-N-Boc-4-(4-fluorophenyl)-4-(aminomethyl)piperidine

To a suspension of Raney Ni (1.4g) and EtOH (10mL) in a Parr bottle was added 1-N-Boc-4-(4-fluorophenyl)-4-cyanopiperidine (4.64g, 15.2mmol) in EtOH (20mL). The solution was bubbled with nitrogen for 5 min, NH₄OH (20mL, 30%) was added. The Parr bottle was shaked with 50 PSI of H₂ at room temperature for 24 hrs. The mixture was filtered through celite and the filtrate was concentrated. The residue was then dissolved in DCM and water. The organic layer was dried, filtered, and concentrated. The residue was purified by chromatography (0-5% MeOH-DCM) to give the title compound as a yellow oil (3.23g, 69% yield). MS m/z 209.34 (M+H-Boc)

### d) 1-N-Boc-4-(4-fluorophenyl)-4-(3-(3-cyanonaphth-1-yl)-(2-azaprop-1-yl))piperidine

To a cooled solution (0°C) containing 1-N-Boc-4-(4-fluorophenyl)-4-(aminomethyl)piperidine (0.258g, 0.84mmol) and triethylamine (0.281mL, 2.02mmol) in DMF (4 mL) was added a solution containing 3-cyano-1-iodomethyl naphthalene (0.245g, 0.84mmol) in DMF (3 mL) over a period of 5 min. The solution was stirred at 0°C for 35 min, then allowed to warm to room temperature overnight. The mixture was partitioned between ethyl acetate and saturated NaHCO₃, the organic layer was removed, and the aqueous layer extracted with ethyl acetate (2x). The organic extracts were washed with saturated brine, dried over MgSO₄, filtered, and concentrated. The residue was purified by chromatography (0-1% MeOH-DCM) to give the title compound as a light yellow oil (0.302g, 76% yield). MS m/z 374.54 (M+H)

### Example 35: 4-(4-Fluorophenyl)-4-(3-(3-cyano-2-ethylnaphth-1-yl)-(2-azaprop-1-yl))piperidine. (M696462)

The title compound of the following structure 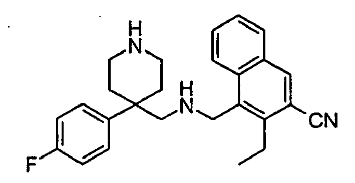 was prepared as a citrate salt in the same manner as Example 34 but with replacement of 3-cyano-1-iodomethyl naphthalene with the 3-cyano-2-ethyl-1-iodomethyl naphthalene, the title compound was obtained as a foaming light yellow solid. MS m/z 402.59 (M+H)

### Example 36: 4-(4-Fluorophenyl)-4-(3-3-cyano-2-methoxynaphth-1-yl)-(2-azaprop-1-yl))piperidine.

The title compound of the following structure 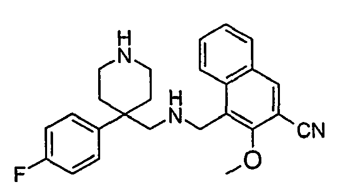 was prepared as a citrate salt in the same manner as Example 34 but with replacement of 3-cyano-1-iodomethyl naphthalene with the 3-cyano-2-methoxy-1-iodomethyl naphthalene, the title compound was obtained as a foaming light yellow solid. MS m/z 404.57 (M+H)

### Example 37: 4-(4-Fluorophenyl)-4-(3-(2,4-dimethoxy-3-cyanonaphth-1-yl)-(2-azaprop-1-yl))piperidine.

The title compound of the following structure 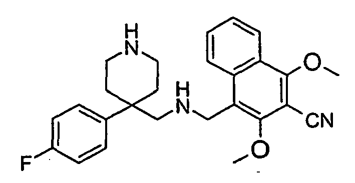 was prepared as a citrate salt in the same manner as Example 34 but with replacement of 3-cyano-1-iodomethyl naphthalene with the 2,4-dimethoxy-3-cyano-1-iodomethyl naphthalene, the title compound was obtained as a foaming light yellow solid. MS m/z 434.57 (M+H)

### Example 38: 4-(4-Fluorolphenyl)-4-(3-(3-cyanonaphth-1-yl)-(2-N-methyl-2-azaprop-1-yl))piperidine.

The title compound of the following structure 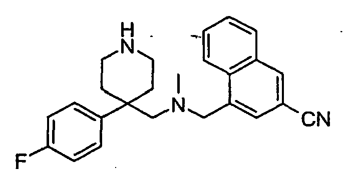 was prepared as a citrate salt as follows. To a solution of 1-N-Boc-4-(4-fluorophenyl)-4-(3-(3-cyanonaphth-1-yl)-(2-N-methyl-2-azaprop-1-yl))piperidine (68mg, 0.14mmol) in DCM (2 mL) was added trifluoroacetic acid (0.22mL, 2.8mmol). The solution was stirred at room temperature for 2 hrs. Solvent was evaporated, EtOAc and saturated NaHCO₃ was added. The organic layer was then washed with saturated NaCl, dried over MgSO₄, filtered and concentrated to give the title product as a foaming light yellow solid (55mg, 100% yield) MS m/z 388.56 (M+H). The citrate salt was obtained by standard procedure.

The requisite 1-N-Boc-4-(4-fluorophenyl)-4-(3-(3-cyanonaphth-1-yl)-(2-N-methyl-2-azaprop-1-yl))piperidine was prepared as follow:

To a solution of 1-N-Boc-4-(4-fluorophenyl)-4-(3-(3-cyanonaphth-1-yl)-(2-azaprop-1-yl))piperidine (0.194g, 0.41mmol) in MeOH (10mL) was added sodium methoxide (116mg, 2.1mmol). The solution was heated to 70°C for 1.5 hrs and paraformaldehyde (38mg, 1.23mmol) was added. The mixture was heated at 70°C for another 5 hrs. After cooled to room temperature, sodium borohydride (31mg, 0.82mmol) was added. The mixture was stirred at room temperature for 24 hrs. EtOAc and saturated NaHCO₃ were added. The organic layer was then washed with saturated NaCl, dried over MgSO₄, filtered and concentrated. The residue was purified by chromatography (0-5% MeOH-DCM) to give the title compound as a colorless oil (68mg, 34% yield). MS m/z 488.58 (M+H).

### Example 39: 4-(4-Fluorophenyl)-4-(3-(3-cyano-2-methoxynaphth-1-yl)-(2-N-methyl-2-azaprop-1-yl))piperidine.

The title compound of the following structure 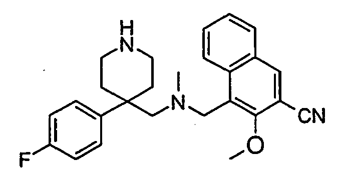 was prepared as a citrate salt in the same manner as Example 38 but with replacement of 1-N-Boc-4-(4-fluorophenyl)-4-(3-(3-cyanonaphth-1-yl)-(2-azaprop-1-yl))piperidine with the 1-N-Boc-4-(4-fluorophenyl)-4-(3-(3-cyano-2-methoxynaphth-1-yl)-(2-azaprop-1-yl))piperidine, the title compound was obtained as a foaming light yellow solid. MS m/z 418.57 (M+H)

### Example 40: 4-(4-Fluorophenyl)-4-(3-(3-cyano-2-ethylnapth-1-yl)-(2-N-methyl-2-azaprop-1-yl))piperidine. (M706931)

The title compound of the following structure 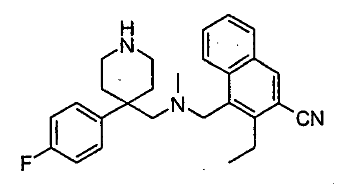 was prepared as a citrate salt in the same manner as Example 38 but with replacement of 1-N-Boc-4-(4-fluorophenyl)-4-(3-(3-cyanonaphth-1-yl)-(2-azaprop-1-yl))piperidine with the 1-N-Boc-4-(4-fluorophenyl)-4-(3-(3-cyano-2-ethylnaphth-1-yl)-(2-azaprop-1-yl))piperidine, the title compound was obtained as a foaming light yellow solid. MS m/z 416.59 (M+H)

### Example 41: 4-(4-Fluorophenyl)-4-(3-(2,4-dimethoxy-3-cyanonaphth-1-yl)-(2-N-methyl-2-azaprop-1-yl))piperidine.

The title compound of the following structure 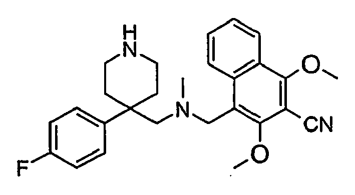 was prepared as a citrate salt in the same manner as Example 38 but with replacement of 1-N-Boc-4-(4-fluorophenyl)-4-(3-(3-cyanonaphth-1-yl)-(2-azaprop-1-yl))piperidine with the 1-N-Boc-4-(4-fluorophenyl)-4-(3-(2,4-dimethoxy-3-cyanonaphth-1-yl)-(2-azaprop-1-yl))piperidine, the title compound was obtained as a foaming light yellow solid. MS m/z 448.57 (M+H)

### Example 42:

Following conventional procedures well known in the pharmaceutical art, the following representative pharmaceutical dosage forms may be prepared containing a compound such as Compound A in accord with formula I:

| Tablet | mg/tablet |
|---|---|
| Compound in accord with structural diagram I | 50.0 |
| Mannitol, USP | 223.75 |
| Croscarmellose sodium | 60 |
| Maize starch | 15 |
| Hydroxypropylmethylcellulose (HPMC), USP | 2.25 |
| Magnesium stearate | 3.0 |

| Capsule | mg/capsule |
|---|---|
| Compound in accord with structural diagram I | 10.0 |
| Mannitol, USP | 488.5 |
| Croscarmellose sodium | 15 |
| Magnesium stearate | 1.5 |

The pharmaceutical dosage form is administered to a patient in need thereof at a frequency depending on the patient and the precise disease condition being treated.

## Claims

1. A compound in accord with formula I: 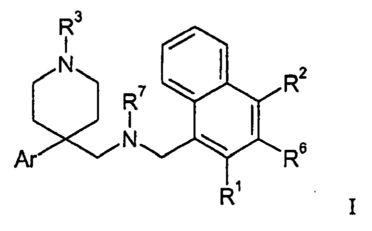 wherein:
R¹ and R² at each occurrence is independently selected from hydrogen, CN, CF₃, OCF₃, OCHF₂, halogen, C₁₋₄ alkyl, C₂₋₄ alkenyl, C₂₋₄ alkynyl, R^{a}, R^{b}, SR^{a}, NR^{a}R^{b}, CH₂NR^{a}R^{b}, OR^{c}, or CH₂OR^{c}, where R^{a}, R^{b}, and R^{c} are independently at each occurrence selected from hydrogen, C₁₋₆ alkyl, C(O)R^{d}, C(O)NHR^{d}, CO₂R^{d}, or R^{a} and R^{b} may together be (CH₂)ⱼG(CH₂)ₖ or G(CH₂)ⱼG where G is oxygen, j is 1, 2, 3 or 4, k is 0, 1 or 2; R^{d} at each occurrence is independently selected from C₁₋₆ alkyl;
R³ is hydrogen or C₁₋₄ alkyl;
R⁶ is hydrogen, CN, C₁₋₄ alkyl or C₁₋₄ alkoxy;
R⁷ is hydrogen or C₁₋₄ alkyl, and
Ar is phenyl or phenyl substituted at one or two positions with moieties independently selected from R⁴ or R⁵ where R⁴ and R⁵ are at each occurrence independently selected from halogen, C₁₋₄ alkoxy or halogenated C₁₋₄ alkyl; and pharmaceutically-acceptable salts thereof.

2. A compound according to Claim 1, in accord with formula II, 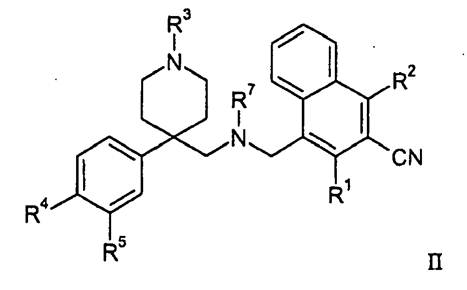 wherein:
R¹, R², R³, R⁴, R⁵ and R⁷ are as defined in Claim 1, and pharmaceutically-acceptable salts thereof.

3. A compound according to Claim 1, wherein:
Ar is selected from 4-chlorophenyl, 4-fluorophenyl, 4-methoxyphenyl, 3,4-dichlorophenyl, 3,4-dimethoxyphenyl, or 4-trifluoromethylphenyl, and pharmaceutically-acceptable salts thereof.

4. A compound according to Claim 1, wherein
R¹ is selected from hydrogen, methoxy or ethyl;
R² is selected from hydrogen or methoxy;
R³ is selected from hydrogen or methyl; and pharmaceutically-acceptable salts thereof.

5. A pharmaceutically-acceptable salts of a compound according to Claim 1 made with an inorganic or organic acid which affords a physiologically-acceptable anion.

6. A pharmaceutically-acceptable salts of a compound according to Claim 5, wherein said inorganic or organic acid is selected from hydrochloric, hydrobromic, sulfuric, phosphoric, methanesulfonic, sulfamic, para-toluenesulfonic, acetic, citric, lactic, tartaric, malonic, fumaric, ethanesulfonic, benzenesulfonic, cyclohexylsulfamic, salicyclic or quinic acids.

7. A pharmaceutical composition comprising a compound according to Claim 1, a pharmaceutically-acceptable salt thereof and a pharmaceutically-acceptable carrier.

8. The use of a compound according to Claim 1 or a pharmaceutically-acceptable salt thereof in the preparation of a medicament for use in a disease condition selected from hypertension, depression in cancer patients, depression in Parkinson's patients, postmyocardial infarction depression, subsyndromal symptomatic depression, depression in infertile women, pediatric depression, major depression, single episode depression, recurrent depression, child abuse induced depression, post partum depression, generalized anxiety disorder, agoraphobia, social phobia, simple phobias, posttraumatic stress syndrome, avoidant personality disorder, premature ejaculation, anorexia nervosa, bulimia nervosa, obesity, addictions to alcohol, cocaine, heroin, phenobarbital, nicotine or benzodiazepines; cluster headache, migraine, pain, Alzheimer's disease, obsessive-compulsive disorder, panic disorder, dementia, amnestic disorders, age-related cognitive decline, dementia in Parkinson's disease, neuroleptic-induced parkinsonism, tardive dyskinesias, hyperprolactinaemia, vasospasm, cerebral vasculature vasospasm, cerebellar ataxia, gastrointestinal tract disorders, negative symptoms of schizophrenia, premenstrual syndrome, fibromyalgia syndrome, stress incontinence, Tourette's syndrome, trichotillomania, kleptomania, male impotence, attention deficit hyperactivity disorder, chronic paroxysmal hemicrania or headache associated with vascular disorders in a mammal, wherein antagonism of the NK₁ receptors and SSRI activity is beneficial.

9. The use according to Claim 8 wherein said compound is administered in combination with a pharmaceutically-acceptable carrier.

## Patentansprüche

1. Verbindung gemäß Formel I: 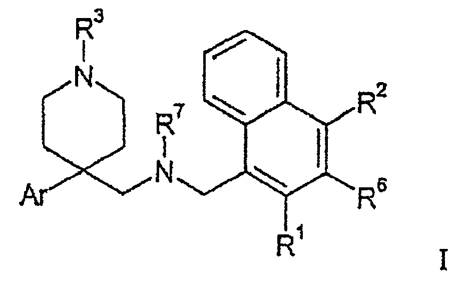 worin:
R¹ und R² jeweils unabhängig voneinander unter Wasserstoff, CN, CF₃, OCF₃, OCHF₂, Halogen, C₁₋₄-Alkyl, C₂₋₄-Alkenyl, C₂₋₄-Alkinyl, R^{a}, R^{b}, SR^{a}, NR^{a}R^{b}, CH₂NR^{a}R^{b}, OR^{c} oder CH₂OR^{c} ausgewählt sind, wobei R^{a}, R^{b} und R^{c} jeweils unabhängig voneinander unter Wasserstoff, C₁₋₆-Alkyl, C(O)R^{d}, C(O)NHR^{d} und CO₂R^{d} ausgewählt sind oder R^{a} und R^{b} gemeinsam für (CH₂)ⱼG(CH₂)ₖ oder G(CH₂)ⱼG stehen können, wobei G für Sauerstoff steht, j für 1, 2, 3 oder 4 steht, k für 0, 1 oder 2 steht; und R^{d} jeweils unabhängig voneinander unter C₁₋₆-Alkyl ausgewählt ist;
R³ für Wasserstoff oder C₁₋₄-Alkyl steht;
R⁶ für Wasserstoff, CN, C₁₋₄-Alkyl oder C₁₋₄-Alkoxy steht;
R⁷ für Wasserstoff oder C₁₋₄-Alkyl steht und
Ar für Phenyl oder in einer oder zwei Positionen mit unabhängig voneinander unter R⁴ oder R⁵ ausgewählten Gruppierungen substituiertes Phenyl steht, wobei R⁴ und R⁵ jeweils unabhängig voneinander unter Halogen, C₁₋₄-Alkoxy oder halogeniertem C₁₋₄-Alkyl ausgewählt sind;
und pharmazeutisch annehmbare Salze davon.

2. Verbindung nach Anspruch 1 gemäß Formel II 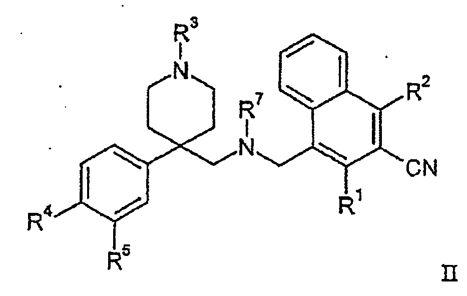 worin:
R¹ R² , R³ , R⁴ , R⁵ und R⁷ die in Anspruch 1 angegebene Bedeutung besitzen,
und pharmazeutisch annehmbare Salze davon.

3. Verbindung nach Anspruch 1, worin:
Ar unter 4-Chlorphenyl, 4-Fluorphenyl, 4-Methoxyphenyl, 3,4-Dichlorphenyl, 3,4-Dimethoxyphenyl oder 4-Trifluormethyl ausgewählt ist,
und pharmazeutisch annehmbare Salze davon.

4. Verbindung nach Anspruch 1, worin:
R¹ unter Wasserstoff, Methoxy oder Ethyl ausgewählt ist;
R² unter Wasserstoff oder Methoxy ausgewählt ist und
R³ unter Wasserstoff oder Methyl ausgewählt ist;
und pharmazeutisch annehmbare Salze davon.

5. Pharmazeutisch annehmbares Salz einer Verbindung nach Anspruch 1, hergestellt mit einer anorganischen oder organischen Säure, die ein physiologisch annehmbares Anion liefert.

6. Pharmazeutisch annehmbares Salz einer Verbindung nach Anspruch 5, worin die anorganische oder organische Säure unter Salzsäure, Bromwasserstoffsäure, Schwefelsäure, Phosphorsäure, Methansulfonsäure, Sulfamidsäure, para-Toluolsulfonsäure, Essigsäure, Citronensäure, Milchsäure, Weinsäure, Malonsäure, Fumarsäure, Ethansulfonsäure, Benzolsulfonsäure, Cyclohexylsulfamidsäure, Salicylsäure und Chinasäure ausgewählt ist.

7. Pharmazeutische Zusammensetzung, enthaltend eine Verbindung nach Anspruch 1 oder ein pharmazeutisch annehmbares Salz davon und einen pharmazeutisch annehmbaren Träger.

8. Verwendung einer Verbindung nach Anspruch 1 oder eines pharmazeutisch annehmbaren Salzes davon bei der Herstellung eines Arzneimittels zur Behandlung eines unter Hypertonie, Depression bei Krebspatienten, Depression bei Parkinsonpatienten, Postmyokardinfarkt-Depression, subsyndromaler symptomatischer Depression, Depression bei unfruchtbaren Frauen, Kinderdepression, Major Depression, einepisodiger Depression, rezividierender Depression, durch Kindesmißbrauch verursachter Depression, postpartaler Depression, generalisierter Angstneurose, Agoraphobie, sozialer Phobie, einfachen Phobien, posttraumatischem Streßsyndrom, ängstlichvermeidender Persönlichkeitsstörung, vorzeitiger Ejakulation, Anorexia nervosa, Bulimia nervosa, Fettleibigkeit, Alkohol-, Kokain-, Heroin-, Phenobarbital-, Nikotin- oder Benzodiazepin-Abhängigkeit, Cluster-Headache, Migräne, Schmerz, Alzheimer-Krankheit, zwangsneurose, Panikstörung, Demenz, amnestischen Störungen, altersbedingtem Nachlassen der kognitiven Fähigkeiten, Demenz bei Parkinsonscher Krankheit, Neuroleptika-induziertem Parkinsonismus, tradiven Dyskinesien, Hyperprolactinämie, Vasospasmus, Vasospasmus des Hirngefäßsystems, zerebellärer Ataxie, Erkrankungen des Magen-Darm-Trakts, negativen Symptomen von Schizophrenie, prämenstruellem Syndrom, Fibromyalgie-Syndrom, Streßinkontinenz, Tourette-Syndrom, Trichotillomanie, Kleptomanie, männlicher Impotenz, Aufmerksamkeitsdefizit-Hyperaktivitätssyndrom, chronischer paroxysmaler Hemikranie oder mit Gefäßerkrankungen assoziierten Kopfschmerzen bei einem Säugetier ausgewählten Krankheitszustands, bei dem Antagonismus der NK₁-Rezeptoren und SSRI-Aktivität vorteilhaft ist.

9. Verwendung nach Anspruch 8, bei der die Verbindung in Kombination mit einem pharmazeutisch annehmbaren Träger verabreicht wird.

## Revendications

1. Composé conformément à la formule I : 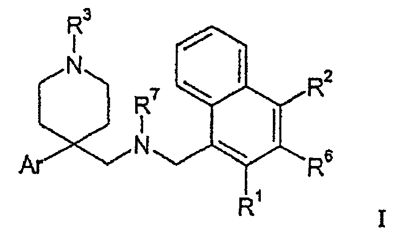 dans laquelle :
R¹ et R² dans chaque cas sont indépendamment choisis parmi l'atome d'hydrogène, les groupes CN, CF₃, OCF₃, OCHF₂, halogène, alkyle en C₁ à C₄, alcényle en C₂ à C₄, alcynyle en C₂ à C₄, R^{a}, R^{b}, SR^{a}, NR^{a}R^{b}, CH₂NR^{a}R^{b}, OR^{c} ou CH₂OR^{c}, dans lesquels R^{a}, R^{b} et R^{c} sont indépendamment dans chaque cas choisis parmi l'atome d'hydrogène, les groupes alkyle en C₁ à C₆, C(O)R^{d}, C(O)NHR^{d}, CO₂R^{d}, ou R^{a} et R^{b} peuvent ensemble être un groupe (CH₂)ⱼG(CH₂)ₖ ou G(CH₂)ⱼG dans lesquels G est un atome d'oxygène, j vaut 1, 2, 3 ou 4, k vaut 0, 1 ou 2 ; R^{d} dans chaque cas est indépendamment choisi à partir d'un groupe alkyle en C₁ à C₆ ;
R³ est un atome d'hydrogène ou un groupe alkyle en C₁ à C₄ ;
R⁶ est un atome d'hydrogène, un groupe CN, un groupe alkyle en C₁ à C₄ ou un groupe alcoxy en C₁ à C₄ ;
R⁷ est un atome d'hydrogène ou un groupe alkyle en C₁ à C₄, et
Ar est un groupe phényle ou phényle substitué à une ou deux positions avec des groupements indépendamment choisis parmi R⁴ ou R⁵, où R⁴ et R⁵ sont dans chaque cas indépendamment choisis parmi un halogène, un groupe alcoxy en C₁ à C₄ ou un groupe alkyle en C₁ à C₄ halogéné ;
et sels pharmaceutiquement acceptables de celui-ci.

2. Composé selon la revendication 1, conformément avec la formule II, 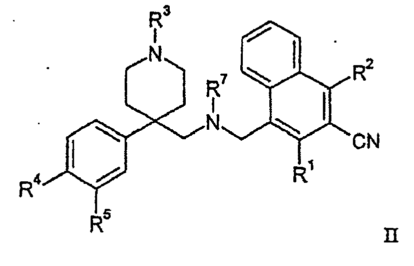 dans laquelle :
R¹, R², R³, R⁴, R⁵ et R⁷ sont tels que définis dans la revendication 1,
et sels pharmaceutiquement acceptables de celui-ci.

3. Composé selon la revendication 1, dans lequel :
Ar est choisi parmi les groupes 4-chlorophényle, 4-fluorophényle, 4-méthoxyphényle, 3,4-dichlorophényle, 3,4-diméthoxyphényle ou 4-trifluorométhylphényle,
et sels pharmaceutiquement acceptables de celui-ci.

4. Composé selon la revendication 1, dans lequel :
R¹ est choisi parmi un atome d'hydrogène, un groupe méthoxy ou un groupe éthyle ;
R² est choisi parmi un atome d'hydrogène ou un groupe méthoxy ;
R³ est choisi parmi un atome d'hydrogène ou un groupe méthyle ;
et sels pharmaceutiquement acceptables de celui-ci.

5. Sels pharmaceutiquement acceptables d'un composé selon la revendication 1, fabriqués avec un acide inorganique ou organique qui fournit un anion physiologiquement acceptable.

6. Sels pharmaceutiquement acceptables d'un composé selon la revendication 5, dans lesquels ledit acide inorganique ou organique est choisi parmi les acides chlorhydrique, bromhydrique, sulfurique, phosphorique, méthanesulfonique, sulfamique, paratoluènesulfonique, acétique, citrique, lactique, tartrique, malonique, fumarique, éthanesulfonique, benzènesulfonique, cyclohexylsulfamique, salicylique ou quinique.

7. Composition pharmaceutique comprenant un composé selon la revendication 1, un sel pharmaceutiquement acceptable de celui-ci et un vecteur pharmaceutiquement acceptable.

8. Utilisation d'un composé selon la revendication 1 ou d'un sel pharmaceutiquement acceptable de celui-ci dans la préparation d'un médicament pour utilisation dans un état pathologique choisi parmi l'hypertension, la dépression chez les patients atteints d'un cancer, la dépression chez les patients atteints de la maladie de Parkinson, la dépression post-infarctus du myocarde, la dépression symptomatique subsyndromale, la dépression chez les femmes stériles, la dépression pédiatrique, la dépression grave, l'épisode dépressif unique, la dépression récidivante, la dépression induite par de mauvais traitements à l'égard d'un enfant, la dépression post-partum, le trouble d'anxiété généralisée, l'agoraphobie, la phobie sociale, les phobies simples, le syndrome du stress post-traumatique, le trouble de la personnalité évitante, l'éjaculation précoce, l'anorexie, la boulimie, l'obésité, les dépendances à l'alcool, à la cocaïne, à l'héroïne, au phénobarbital, à la nicotine ou aux benzodiazépines ; la céphalée vasculaire de Horton, la migraine, la douleur, la maladie d'Alzheimer, le trouble obsessionnel compulsif, le trouble panique, la démence, les troubles d'amnésie, le déclin cognitif lié à l'âge, la démence dans la maladie de Parkinson, le parkinsonisme neuroleptique, la dyskinésie tardive, l'hyperprolactinémie, le vasospasme, le vasospasme du système vasculaire cérébral, l'ataxie cérébelleuse, les troubles du tractus gastro-intestinal, les symptômes négatifs de schizophrénie, le syndrome prémenstruel, le syndrome de fibromyalgie, l'incontinence d'effort, le syndrome de Tourette, la trichotillomanie, la kleptomanie, l'impuissance, le déficit de l'attention, le trouble d'hyperactivité, l'hémicrânie paroxystique chronique ou les maux de tête associés aux désordres vasculaires chez un mammifère, dans laquelle l'antagonisme des récepteurs NK₁ et de l'activité de l'ISRS est bénéfique.

9. Utilisation selon la revendication 8, dans laquelle ledit composé est administré en association avec un vecteur pharmaceutiquement acceptable.
